(19) Europäisches Patentamt / European Patent Office / Office européen des brevets

(11) **EP 2 918 549 A1**

(12) **EUROPÄISCHE PATENTANMELDUNG**

(43) Veröffentlichungstag:
**16.09.2015 Patentblatt 2015/38**

(21) Anmeldenummer: **14159871.4**

(22) Anmeldetag: **14.03.2014**

(51) Int Cl.:
*C02F 1/02* (2006.01)   *B09B 3/00* (2006.01)
*B01J 3/00* (2006.01)   *B01J 4/00* (2006.01)
*C02F 11/04* (2006.01)   *C02F 11/08* (2006.01)
*C02F 1/52* (2006.01)   *C02F 101/10* (2006.01)
*B01D 9/00* (2006.01)   *C02F 103/26* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Paul Scherrer Institut**
**5232 Villigen (CH)**

(72) Erfinder:
• **De Boni, Erich**
**5400 Baden (CH)**

• **Reimer, Joachim**
**5430 Wettingen (CH)**
• **Peng, Gaël**
**2740 Moutier (CH)**
• **Zöhrer, Hemma**
**6260 Bruck am Ziller (AT)**
• **Vogel, Frédéric**
**5018 Erlinsbach (CH)**

(74) Vertreter: **Fischer, Michael**
**Siemens AG**
**Postfach 22 16 34**
**80506 München (DE)**

(54) **Salzabscheider und Verfahren zur Erzeugung eines methanhaltigen Gasgemisches aus Biomasse unter Einsatz eines Salzabscheiders**

(57) Erfindungsgemäss ist Salzabscheider (2) zum Abscheiden von Salzen und/oder Feststoffen aus einem pumpfähigen wässrigen Fluidgemisch unter Verfahrensbedingungen, die vorzugsweise im Wesentlichen im Bereich des kritischen Punktes für das Fluidgemisch liegen, offenbart, wobei der Salzabscheider die folgenden Komponenten umfasst:

a) eine Reaktionszone in Form eines Hohlraums (6) zur Überführung des pumpfähigen wässrigen Fluidgemisches in ein Rohgemisch für eine Weiterverarbeitung, z.B. eine Methanierungsreaktion;

b) eine Zuführungsöffnung (8) für das pumpfähige wässrige Fluidgemisch zu dem Hohlraum (6), wobei die Zuführungsöffnung (8) in einem Steigrohr (10) realisiert ist, das in den Hohlraum (6) hineinragt;

c) eine erste Abzugsöffnung (12) für das von Salzen und/oder Feststoffen befreite Rohgemisch, wobei die erste Abzugsöffnung (12) im oberen Bereich des Hohlraums (6) angeordnet ist; und

d) eine zweite Abzugsöffnung (16) für eine die Salze und/oder die Feststoffe umfassende Sole, wobei die zweite Abzugsöffnung (16) im unteren Bereich des Hohlraums (6) angeordnet ist und tiefer liegt als die Zuführungsöffnung (8).

FIG 1

**Beschreibung**

Die vorliegende Erfindung betrifft einen Salzabscheider sowie ein Verfahren zur Erzeugung eines methanhaltigen Gasgemisches aus Biomasse unter Einsatz eines Salzabscheiders.

Unter dem Begriff "Biomasse" ist pflanzliches oder tierisches Material zu verstehen. Beispielhaft können Holz, Mist, Gülle, Stroh, Gras, Algen, Klärschlamm und Schlachtabfälle genannt werden. Das vorliegende Verfahren eignet sich aber auch für andere Stoffe mit organischen Anteilen, wie z.B. Kunststoffabfälle, Abwässer, Kehricht, Altreifen, Altpapier, Altöle, organische Lösungsmittel, fossile Biomasse (Torf, Kohle, Erdöl). Der Salzabscheider eignet sich ganz allgemein zur Abscheidung von Salzen aus wässrigen Lösungen mit und ohne Organik.

In einer vom Bundesamt für Energie (BFE, Schweiz) in Auftrag gegebenen Studie der Hochschule Wädenswil, "Scheurer, K.; Baier, U. Biogene Güter in der Schweiz. Massen- und Energieflüsse. Hochschule Wädenswil, im Auftrag des BFE, Programm Biomasse, Schlussbericht, Februar 2001", wird auf das grosse, weitgehend ungenutzte energetische Potential von Gülle hingewiesen. Der gesamte Hofdüngeranfall (Mist + Gülle) betrug 1998/99 2.283 Mio t TS (Trockensubstanz), was einem Energieinhalt von 37 PJ entspricht. Die Vergärung von 4'700 t TS Hofdünger lieferte 1998 rund 48 TJ an Energie in Form von Biogas, was nur ca. 0.1% des gesamten Energiepotentials im Hofdünger darstellt. Bei der Vergärung fallen zudem grössere Mengen nicht vergärbaren Feststoffs an. Holzartige Biomasse kann praktisch nicht vergärt werden.

Im Folgendem bezeichnet der Begriff "hydrothermal" ein wässriges System unter erhöhtem Druck und erhöhter Temperatur, typischerweise in der Nähe oder über dem kritischen Punkt von Wasser (374°C, 221 bar). Nahekritisches und überkritisches Wasser bilden ein interessantes Reaktionsmedium, um chemische Reaktionen durchzuführen. Insbesondere eignet sich dieses Medium für die Hydrolyse und die Umsetzung von Biomasse zu flüssigen und gasförmigen Produkten. Da der Übergang eines flüssigen Systems unter Druck ins Überkritische keinen echten Phasenübergang darstellt, muss für das in der Biomasse enthaltene Wasser keine Verdampfungsenthalpie aufgewendet werden, was im Gegensatz zu Gasphasenprozessen (z.B. atmosphärische Vergasung nasser Biomasse) steht. Daher haben hydrothermale Prozesse das Potential für hohe thermische Wirkungsgrade.

Die meist bevorzugte Reaktion für die Umwandlung von Biomasse zu Methan kann beispielhaft für Holz mit folgender Stöchiometrie beschrieben werden:

$$CH_{1.52}O_{0.64}(s) \ + \ 0.3 \ H_2O(g) \ \rightarrow \ 0.53 \ CH_4(g) \ + \ 0.47 \ CO_2(g) \qquad (1)$$

Unter normalen Bedingungen (geringer Wasser-Partialdruck) verläuft die Umsetzung von Biomasse mit Wasser nicht bzw. nicht vollständig nach Gl. (1), sondern es entstehen Nebenprodukte wie z.B. Teere oder fester Kohlenstoff (Koks). Gelingt es, die Reaktionsbedingungen so zu wählen, dass Reaktion (1) vollständig abläuft, kann ein hoher thermischer Wirkungsgrad erwartet werden, da die Reaktion (1) leicht exotherm ist. Der theoretisch maximal mögliche Wirkungsgrad beträgt 95% (bezogen auf den unteren Heizwert $H_u$ des Holzes). Eine von der Anmelderin durchgeführte Systemanalyse für einen kommerziellen Prozess ergab einen erzielbaren Wirkungsgrad im Bereich von 70-80% für Holz. Dieses wurde ausführlich in der Literaturstelle "Vogel, F., and F. Hildebrand, Catalytic Hydrothermal Gasification of Woody Biomass at High Feed Concentrations. Chem. Eng. Trans. 2, 2002, 771-777" beschrieben. Dies ist deutlich höher als der Wirkungsgrad anderer Verfahren zur Umwandlung von Holz zu Methan. Zusammengefasst bleiben die derzeit bekannten Prozesse zur Methanerzeugung aus Biomasse jedoch hinsichtlich der erreichbaren Wirkungsgrade hinter den theoretischen Erwartungen zurück, so dass sie derzeit nicht wirtschaftlich eingesetzt werden können.

Zur Verbesserung des Wirkungsgrades eines hydrothermalen Prozesses zur Methanerzeugung ist in der europäischen Patentanmeldung EP 1 772 202 A1 ein Verfahren zur Erzeugung von Methan aus Biomasse offenbart, das die nachfolgenden Verfahrensschritte aufweist:

a) aus der Biomasse wird ein Biomassebrei unter Einstellung eines optimalen Trockenmasseanteils hergestellt,
b) der Biomassebrei wird unter Druck gesetzt,
c) zur Verflüssigung der festen organischen Bestandteile des Biomassebreis wird der Biomassebrei unter Druck erhitzt,
d) der so unter Druck gesetzte und erhitzte Biomassebrei wird weiter auf mindestens die der Mischung eigene kritische Temperatur erhitzt,
e) unter Druck und erhöhter Temperatur werden dabei ausgefällte Feststoffe von der fluiden Phase abgetrennt, und
f) mittels eines katalytischen Reaktors wird unter Druck und erhöhter Temperatur mindestens ein Teil der fluiden Phase zu einem methanreichen Gas vergast.

Auf diese Weise wurde ein Verfahren geschaffen, welches einen sehr hohen Wirkungsgrad aufweist, weil ein Grossteil der die katalytische Vergasung störenden Stoffe, im besonderen Salze, durch Ausfällung unter überkritischen Bedingungen von der Mischung abgetrennt werden können. Auf diese Weise kann für die katalytische Vergasung eine hohe Ausbeute an Methan und eine hohe Reaktionsrate bei gleichzeitig hoher Standzeit des Katalysators erzielt werden.

Umfangreiche Aufsätze in der Literatur zeigen dabei, dass der Salzabscheidung in diesem hydrothermalen Prozess eine wichtige Bedeutung für die erreichbaren Wirkungsgrade des Gesamtprozesses und für erreichbare Standzeit des Methanierungskatalysators zukommt. Dennoch haftet allen bislang bekannten Salzabscheidern der Nachteil an, dass die Salzabscheidung entweder immer noch nicht zufriedenstellend ist oder aber zufriendenstellend ist, jedoch erhöhte thermodynamische oder mechanisch-konstruktive Aufwendungen erforderlich sind. Weiter stellen auch Verstopfungen und Ablagerungen in derartigen Salzabscheidern ein grosses Problem dar. Im Besonderen hat es sich gezeigt, dass die bekannten Salzabscheider besonders bei der superkritischen Oxidation von Wasser (Super Critical Water Oxidation) nicht gut funktionieren.

Ausgehend von diesem Stand der Technik liegt der Erfindung daher die Aufgabe zugrunde, einen Salzabscheider und ein Verfahren zur hydrothermalen Generierung eines methanhaltigen Gases aus Biomasse unter Verwendung eines Salzabscheiders anzugeben, wobei der Salzabscheider einfach aufgebaut und zu betreiben sein soll und wobei das Verfahren einen besonders hohen Wirkungsgrad aufweisen soll.

Diese Aufgabe wird bezüglich des Salzabscheiders erfindungsgemäss durch einen Salzabscheider zum Abscheiden von Salzen und/oder Feststoffen aus einem pumpfähigen wässrigen Fluidgemisch unter Verfahrensbedingungen, die vorzugsweise im Wesentlichen im Bereich des kritischen Punktes für Wasser liegen, gelöst, wobei der Salzabscheider die folgenden Komponenten umfassend:

a) eine Reaktionszone in Form eines Hohlraums zur Überführung des pumpfähigen wässrigen Fluidgemisches in ein Rohgemisch für eine nachfolgende Weiterverarbeitung, z.B. eine Methanierungsreaktion;
b) eine Zuführungsöffnung für das pumpfähige wässrige Fluidgemisch zu dem Hohlraum, wobei die Zuführungsöffnung in einem Steigrohr realisiert ist, das in den Hohlraum hineinragt;
c) eine erste Abzugsöffnung für das von Salzen und/oder Feststoffen befreite Rohgemisch, wobei die erste Abzugsöffnung im oberen Bereich des Hohlraums angeordnet ist; und
d) eine zweite Abzugsöffnung für eine die Salze und/oder die Feststoffe umfassende Sole, wobei die zweite Abzugsöffnung im unteren Bereich des Hohlraums angeordnet ist und tiefer liegt als die Zuführungsöffnung.

Überraschenderweise gelingt es mit diesem Salzabscheider die Salze und/oder Feststoffe, die beim Eintreten des pumpfähigen wässrigen Fluidgemisches darin enthalten sind, mit einer bisher noch nicht gekannten Effizienz abzuscheiden und aus dem weiteren Verfahrensstrom zu entfernen. Geeignete Fluidgemische sind hierbei zum Beispiel ein pumpfähiger Biomassebrei, Geothermieabwässer, Abwässer aus Erdölbohrlöchern sowie generell alle Typen von salzhaltigen Prozesswässern. Zum Beispiel ist es mit diesem Design für den Salzabscheider ohne Verstopfung möglich eine Mischung von 100 Millimolar Natriumsulfat und 50 Millimolar Kaliumsulfat abzuscheiden, was bei den bisher bekannten Salzabscheidern regelmässig zu einem festen Niederschlag und damit zu einer Salzansammlung führte, die den Salzabscheider verstopfen kann.

In einer vorteilhaften Ausgestaltung der vorliegenden Erfindung kann es vorgesehen sein, dass der Hohlraum zylinderförmig ausgestaltet und im Wesentlichen senkrecht ausrichtbar ist, wobei die Ausrichtung in senkrechter Richtung grösser als der Durchmesser des Hohlraums ist. Damit hat der Hohlraum die Form einer Steigkolonne, in der das zunehmend vom Salz befreite Rohgemisch aufsteigen und für den nachfolgenden Verarbeitungsprozess abgezogen werden kann. Typischerweise ist es dann auch zweckmässig, wenn die erste Abzugsöffnung im Bereich des höchsten Punktes des Hohlraums angeordnet ist.

Entsprechend ist es ebenso zweckmässig, wenn die zweite Abzugsöffnung im Bereich des tiefsten Punktes des Hohlraums angeordnet ist. Ein Abzug der salz- und/oder Feststoffhaltigen Sole kann dann seitlich erfolgen, sodass die Zuführung des pumpfähigen wässrigen Fluidgemisches direkt von unten senkrecht hinein in den Hohlraum erfolgen kann. In einer weiteren zweckmässigen Ausgestaltung der vorliegenden Erfindung kann dann auch vorgesehen sein, dass sich die Zuführungsöffnung am hohlraumseitigen Ende eines Steigrohres befindet, welches in den Hohlraum senkrecht hineinragt. Auf diese Weise ergibt auch eine genügend grosse räumliche Trennung von Zuführungsöffnung und zweiter Abzugsöffnung, die in einem oberhalb der zweiten Abzugsöffnung liegenden Sumpf resultiert.

Um die Verfahrensbedingungen, die im Wesentlichen im Bereich und vorzugsweise oberhalb des pseudokritischen Punktes für das jeweilige Fluidgemisch liegen sollen, besonders gut einhalten zu können, kann es vorgesehen sein, dass der Hohlraum beheizbar ausgeführt ist. So können beispielsweise auf den Wandungen des Hohlraum angeordnete elektrische Widerstandsheizelemente und/oder auch Induktionsheizelemente vorgesehen sein. Möglich ist auch eine Beheizung der Aussenwand durch heisse Gase, wie z.B. Abgase aus einer Feuerung oder Prozessabgase. Weiterhin ist es möglich, die benötigte Erhitzung durch Zugabe von Oxidationsmitteln im eintretenden Fluid, z.B. Nitrate, Sauerstoff oder Wasserstoffperoxid, zu erreichen.

Bezüglich des Verfahrens wird die oben genannte Aufgabe erfindungsgemäss durch ein Verfahren zur Generierung eines methanhaltigen Gasgemisches aus Biomasse gelöst, bei dem aus einem pumpfähigen wässrigen Biomassebrei vor der Methanierungsreaktion in einem Salzabscheider, der gemäss einem der Ansprüche 1 bis 5 ausgestaltet ist, Salze und/oder Feststoffe entzogen werden.

Vorteilhafte Ausgestaltungen der vorliegenden Erfindung werden nachfolgend anhand der Zeichnung für den Salzabscheider und das mit ihm beispielhaft durchgeführte Verfahren für die Vergasung von Biomasse (z.B. Holz oder Gülle-Feststoff) näher erläutert. Dabei zeigen:

Figur 1    in schematischer Ansicht einen Längsschnitt durch einen Salzabscheider; und

Figur 2    einen zeitlichen Verlauf der spezifischen Leitfähigkeit eines Edukts von 10 wt% Iso-Propanol in Wasser mit einer Salzladung von 0.1 mol/l Natriumsulfat und 0.05 mol/l Kaliumsulfat in einem Salzabscheider gemäss Figur 1 bei einer Temperatur von 450°C und einem Druck von 300 bar.

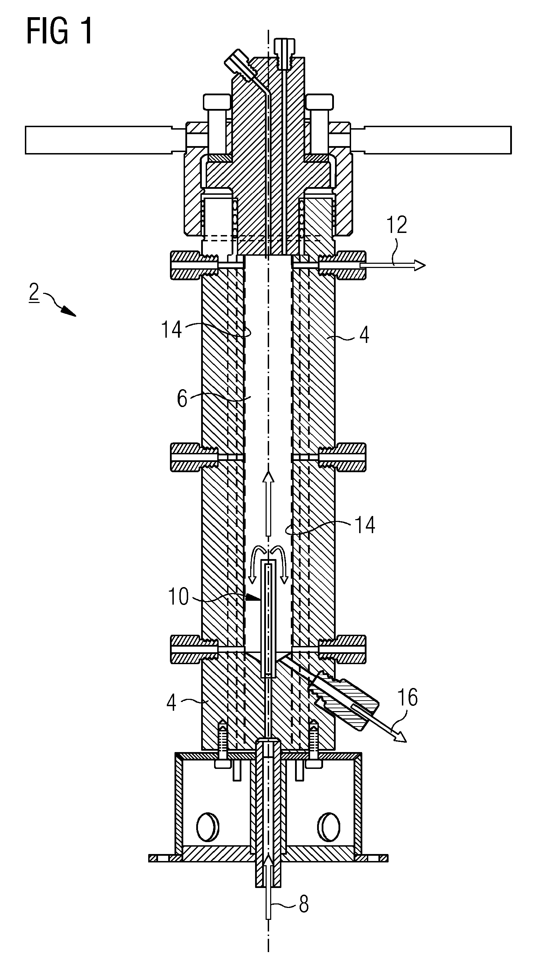

Figur 1 zeigt in schematischer Ansicht einen Längsschnitt durch einen Salzabscheider 2, wie er zur Abtrennung von Salzen und/oder Feststoffe aus einem wässrigen Fluidgemisch eingesetzt wird. Das von Salzen und/oder Feststoffen befreite Rohprodukt wird dann einer Weiterverarbeitung, z.B. einer Methanierungsreaktion, zugeführt, wobei aufgrund der eliminierten Salz- und/oder Feststoffanteile eine hohe Selektivität und Standzeit allfällig eingesetzter Katalysatoren und eine geringe Korrosion der dem Prozess ausgesetzten Oberfläche erreicht werden.

Der Salzabscheider 2 ist typischerweise aus einem Edelstahlgehäuse 4 (oder einem anderen geeigneten Werkstoff wie Titan oder einer Nickellegierung) aufgebaut, das einen zylinderförmigen Hohlraum 6 umschliesst. Der Hohlraum 6 ist in diesem Sinne ein Reaktionsraum, in dem in dem wässrigen Fluidgemisch gelöste Salze unter den im Hohlraum 6 herrschenden thermodynamischen Bedingungen, die im Wesentlichen dem kritischen Punkt für das Fluidgemisch entsprechen, ausgefällt werden können. Im unteren Bereich des Hohlraum 6 ist eine Zuführung 8 vorgesehen, durch die das wässrige Fluidgemisch unter hohem Druck von 200 bis 400 bar bei einer Temperatur von rund 350 bis 500°C in den Hohlraum 6 eingeführt wird. Dabei tritt das wässrige Fluidgemisch an erhöhter Position aus einem Steigrohr 10 in den Hohlraum 6 aus. Im Wesentlichen an der höchsten Position im Hohlraum 6 ist eine erste Abzugsöffnung 12 für ein von Salzen und/oder Feststoffen weitgehend befreites Rohfluid, das dann der eigentlichen Weiterverarbeitung, z.B. einer Methanierungsreaktion, unter Erzielung der o.g. Vorteile zugeführt werden kann. Im Wesentlichen an der tiefsten Position im Hohlraum 6 ist eine zweite Abzugsöffnung 16 für eine salz- und/oder feststoffbeladene Sole vorgesehen, die damit aus dem weiteren Verarbeitungsprozess ausgetragen wird. Zur Aufrechterhaltung der hohen Temperaturen im Hohlraum 6 sind an den Wandungen des Hohlraum angeordnete Heizelemente 14 in Form von Widerstands- und/oder Induktionsheizkörpern vorgesehen. Möglich ist aber auch alternativ oder zusätzlich eine Beheizung der Aussenwand durch heisse Gase, wie z.B. Abgase aus einer Feuerung oder Prozessabgase. Weiterhin ist es möglich, die benötigte Erhitzung durch Zugabe von Oxidationsmitteln im eintretenden Fluid, z.B. Nitrate, Sauerstoff oder Wasserstoffperoxid, zu erreichen.

In vollkommen überraschender Weise hat die Einführung des wässrigen Fluidgemisches von unten her in den Salzabscheider 2 das erfreuliche Resultat einer weitgehenden Abscheidung von Salzen und/oder Festkörper aus dem dann für die Weiterverarbeitung vorgesehenen Rohfluid geführt.

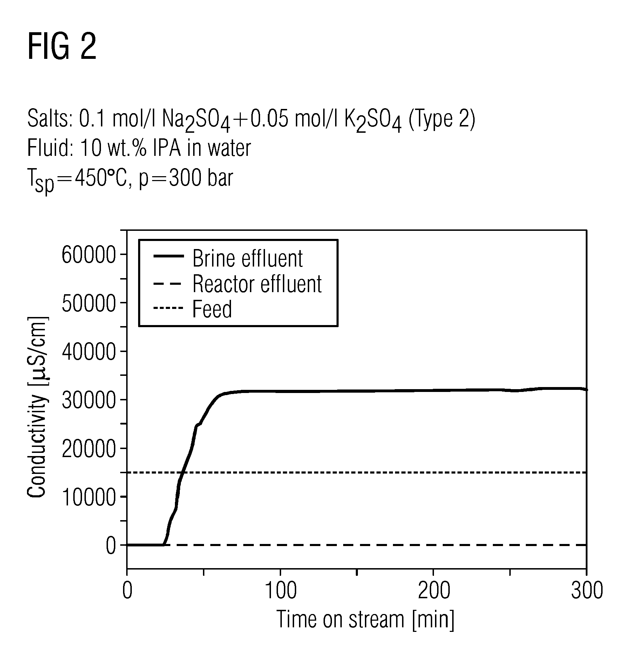

Die Figur 2 zeigt denn auch beispielhaft einen zeitlichen Verlauf der spezifischen Leitfähigkeit eines Edukts von 10 wt% Iso-Propanol in Wasser mit einer Salzladung von 0.1 mol/l Natriumsulfat und 0.05 mol/l Kaliumsulfat in einem Salzabscheider 2 gemäss Figur 1 bei einer Temperatur von 450°C und einem Druck von 300 bar. An dieser Stelle wurde bewusst Isopropanol gewählt, da dieser die Organik eines verflüssigten Biomassebreis gut nachbildet, die bei der Abscheidung von Salzen ein Rolle spielt. Wie in der Figur gezeigt, steigt die Leitfähigkeit der Sole sehr schnell auf etwa 30 mS/cm an. Zugleich ist die Leitfähigkeit des "gereinigten" Rohfluids nahe Null, was auf eine vollständige Abscheidung der die Leitfähigkeit verursachenden Sulfate aus dem Rohfluid schliessen lässt.

Mit Bezug auf die bereits eingangs genannte europäische Patentanmeldung EP 1 772 202 A1 soll auch das Verfahren zur Methanerzeugung nochmals kurz dargestellt werden:

• Die Biomasse wird in einem **1. Prozessschritt** konditioniert, d.h. zerkleinert und auf den gewünschten Trockenmassenanteil (TM) gebracht, vorzugsweise durch Nassmahlung. Dadurch entsteht ein pumpfähiger Brei. Zur Verbesserung der Pumpfähigkeit können der Biomasse andere Zusatzstoffe (z.B. Stärke, Altöle) zugegeben werden. Der erwünschte Trockenmassenanteil beträgt 5 bis 80 Massenprozent, vorzugsweise etwa 15 bis 40 Massenprozent. Das Verfahren arbeitet besonders wirtschaftlich, wenn der organische Trockenmassenanteil etwa 20 Massenprozent und mehr beträgt.

• Der konditionierte Biomassebrei wird in einem **2. Prozessschritt** auf hohen Druck (200-400 bar) gebracht und kontinuierlich oder stossweise gefördert. Als Förderaggregate eignen sich insbesondere Extruder, Hochdruck-Exzenterschneckenpumpen, Kolbenmembranpumpen, und Feststoffpumpen.

- In einem **3. Prozessschritt** wird der Biomassebrei unter Druck auf 200-350°C erhitzt. Dabei verflüssigen sich die festen organischen Biomassebestandteile weitgehend. Zur besseren Erhitzung und Verflüssigung kann diese

Prozessstufe statische Mischelemente und/oder einen Katalysator (z.B. Zinkoxid) enthalten.

- In einem **4. Prozessschritt** wird der unter Druck stehende, erhitzte und verflüssigte Biomassebrei in den Salzabscheider 2 schnell auf ein noch höheres Temperaturniveau gebracht, vorzugsweise im Bereich oder über der kritischen Temperatur der jeweiligen Mischung. Als Anhaltspunkt dient hier die kritische Temperatur von Wasser bei 374°C und 221 bar. Dies kann durch externen Wärmeeintrag (z.B. durch einen Brenner/katalytischen Brenner, der mit zurückgeführtem Produktgas gespiesen wird) oder durch Zugabe von geeigneten Oxidationsmitteln (z.B. Sauerstoff, Luft, Wasserstoffperoxid, Ammonium- und andere Nitrate) direkt in die 4. Prozessstufe (oder eine der vorangehenden Prozessstufen 1-3) geschehen. Dadurch fallen die meisten Salze und restlichen Feststoffe aus und können gesammelt werden. Die gesammelten Ausfällungen werden kontinuierlich oder periodisch durch die zweite Abzugsöffnung 16 aus dem Prozess ausgetragen. Das Abtrennen und Zurückgewinnen von Feststoffen als Salze vor dem katalytischen Vergasungsreaktor unter hydrothermalen Bedingungen sowie die mögliche Zugabe von salzartigen Oxidationsmitteln (Nitrate, z.B. Ammoniumnitrat) zur partiellen Oxidation der Biomasse unter hydrothermalen Bedingungen verbessern die Durchführung und erhöhen die Effizienz des Verfahrens wesentlich. Die abgezogenen Feststoffe sind aufgrund der Beschaffenheit der Ausgangsstoffe sehr reich an Stickstoff-, Phosphor- und Kaliumsalzen und eignen sich daher hervorragend zur Weiterverwendung als Dünger beispielsweise für die Landwirtschaft oder für die Algenzucht.

- In einem **5. Prozessschritt** gelangt der heisse Biomassestrom (das heisse Rohfluid), nunmehr von den meisten Feststoffen befreit, in einen mit einem geeigneten Katalysator beschickten Reaktor, wo die Vergasung zu Methan, Kohlendioxid, Wasserstoff und Spuren von Kohlenmonoxid sowie höheren Kohlenwasserstoffen (Ethan, Propan) stattfindet. Der Katalysator umfasst dabei vorzugsweise Ruthenium und kann daneben auch Nickel (z.B. Raney® Nickel) sowie weiter auch noch Anteile von Chrom und/oder Kupfer aufweisen. Andere Katalysatoren, die auf Ni, Re, oder Rh als aktives Metall basieren, sind ebenfalls einsetzbar. Der Reaktor wird vorzugsweise als Wirbelschichtreaktor, als Monolithreaktor oder als Wandreaktor (mit Katalysator beschichtetes Rohr oder Rohrbündel) ausgestaltet. Es könnten aber auch Rohre verwendet werden, in welche katalytisch beschichtete Bleche eingesetzt sind.

- In einem **6. Prozessschritt** wird der methanreichen Produktstrom dann seiner weiteren Verwendung zugeführt. Dieser Prozessschritt kann ebenfalls dazu benutzt werden, Methan vom $CO_2$ und den restlichen Gaskomponenten zu trennen. So kann der Produktstrom auch auf ca. 50°C gekühlt und die Gasphase unter Druck von der Flüssigphase getrennt werden. In einem geeigneten Apparat (z.B. Waschkolonne, Membrantrennung, Adsorber) kann das Methan von den anderen Komponenten aus der Gasphase abgetrennt werden und steht dann unter hohem Druck (ca. 200 bis 400 bar) zur Verfügung. Dadurch entfällt ein Kompressionsschritt, um das Methan in Gasflaschen abzufüllen, als Treibstoff an einer Gastankstelle anzubieten oder ins Erdgasnetz einzuspeisen. Es ist ebenfalls denkbar, das komprimierte Gas direkt als Brennstoff in einem Gasturbinenprozess zu nutzen.

Hiernach bietet das Bereitstellen von Methan aus Biomasse unter anderem für Erdgastankstellen und/oder für die Einspeisung ins Erdgasnetz, für die Abfüllung in Flaschen, oder die Verwendung als Brennstoff in Gasturbinen geeigneten Druck eine starke wirtschaftliche Verwertbarkeit.

Auch wenn hier die Beschreibung eines Verfahrens zur Gewinnung von Methan aus Biomasse im Vordergrund steht, kann der erfindungsgemässe Salzabscheider auch in Verfahren zur Reinigung von anderen wässrigen Fluidgemischen verwendet werden. Geeignete Fluidgemische sind hierbei zum Beispiel ein pumpfähiger Biomassebrei, Geothermieabwässer, Abwässer aus Erdölbohrlöchern sowie generell alle Typen von salzhaltigen Prozesswässern, mit und ohne Organik.

**Patentansprüche**

1. Salzabscheider (2) zum Abscheiden von Salzen und/oder Feststoffen aus einem pumpfähigen wässrigen Fluidgemisch unter Verfahrensbedingungen, die vorzugsweise im Wesentlichen im Bereich des kritischen Punktes für das Fluidgemisch liegen, umfassend:

   a) eine Reaktionszone in Form eines Hohlraums (6) zur Überführung des pumpfähigen wässrigen Fluidgemisches in ein Rohgemisch für eine Weiterverarbeitung, z.B. eine Methanierungsreaktion;

b) eine Zuführungsöffnung (8) für das pumpfähige wässrige Fluidgemisch zu dem Hohlraum (6), wobei die Zuführungsöffnung (8) in einem Steigrohr (10) realisiert ist, das in den Hohlraum (6) hineinragt;

c) eine erste Abzugsöffnung (12) für das von Salzen und/oder Feststoffen befreite Rohgemisch, wobei die erste Abzugsöffnung (12) im oberen Bereich des Hohlraums (6) angeordnet ist; und

d) eine zweite Abzugsöffnung (16) für eine die Salze und/oder die Feststoffe umfassende Sole, wobei die zweite Abzugsöffnung (16) im unteren Bereich des Hohlraums (6) angeordnet ist und tiefer liegt als die Zuführungsöffnung (8).

2. Salzabscheider (2) nach Anspruch 1,
**dadurch gekennzeichnet, dass**
der Hohlraum (6) zylinderförmig ist und im Wesentlichen senkrecht ausrichtbar ist, wobei die Ausrichtung in senkrechter Richtung grösser als der Durchmesser des Hohlraums (6) ist.

3. Salzabscheider (2) nach Anspruch 1 oder 2,
**dadurch gekennzeichnet, dass**
die erste Abzugsöffnung (12) im Bereich des höchsten Punktes des Hohlraums (6) angeordnet ist.

4. Salzabscheider (2) nach einem der Ansprüche 1 bis 3,
**dadurch gekennzeichnet, dass**
die zweite Abzugsöffnung (16) im Bereich des tiefsten Punktes des Hohlraums (6) angeordnet ist.

5. Salzabscheider (2) nach einem der voranstehenden Ansprüche, **dadurch gekennzeichnet, dass** sich die Zuführungsöffnung (8) am hohlraumseitigen Ende eines Steigrohres (10) befindet, welches senkrecht in den Hohlraum (6) hineinragt.

6. Salzabscheider (2) nach einem der vorangehenden Ansprüche,
**dadurch gekennzeichnet, dass**
der Hohlraum mittels Heizelementen beheizbar ist.

7. Verfahren zur Generierung eines methanhaltigen Gasgemisches aus Biomasse, bei dem aus einem pumpfähigen wässrigen Biomassebrei vor der Methanierungsreaktion in einem Salzabscheider (2) gemäss einem der Ansprüche 1 bis 6 Salze und/oder Feststoffe abgezogen werden.

FIG 1

# FIG 2

Salts: 0.1 mol/l $Na_2SO_4$ + 0.05 mol/l $K_2SO_4$ (Type 2)
Fluid: 10 wt.% IPA in water
$T_{sp}$ = 450°C, p = 300 bar

Europäisches
Patentamt

European
Patent Office

Office européen
des brevets

**EUROPÄISCHER RECHERCHENBERICHT**

Nummer der Anmeldung

EP 14 15 9871

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 102 17 165 A1 (KARLSRUHE FORSCHZENT [DE]) 5. Februar 2004 (2004-02-05) * Absatz [0002]; Ansprüche 1-5 * ----- | 1 2-7 | INV. C02F1/02 B09B3/00 B01J3/00 |
| Y | | | B01J4/00 |
| Y | DE 202 20 307 U1 (KARLSRUHE FORSCHZENT [DE]) 30. April 2003 (2003-04-30) * Absatz, der Seiten 1 und 2 der Beschreibung überbrückt.; Ansprüche 1-6; Abbildung 1 * ----- | 2-6 | C02F11/04 C02F11/08 ADD. C02F1/52 |
| Y | WO 2011/154226 A1 (SCHERRER INST PAUL [CH]; GASSNER MARTIN [CH]; MARECHAL FRANCOIS [CH];) 15. Dezember 2011 (2011-12-15) * Seite 4, Zeile 19 - Seite 4, Zeile 35; Abbildung 1 * ----- | 2-6 | C02F101/10 B01D9/00 C02F103/26 |
| A | DE 299 13 370 U1 (KARLSRUHE FORSCHZENT [DE]) 23. September 1999 (1999-09-23) * Anspruch 1; Abbildung 1 * ----- | 1-6 | |
| A | US 5 707 417 A (YOKOYAMA SHINYA [JP] ET AL) 13. Januar 1998 (1998-01-13) * Spalte 3, Zeile 17 - Spalte 3, Zeile 46; Ansprüche 1-3; Abbildung 1 * ----- | 1-7 | RECHERCHIERTE SACHGEBIETE (IPC) C02F B01J C12P B09B B01D |
| Y | WO 2013/005202 A1 (HOLLINGFORD LTD [IE]; O'REGAN JOHN [IE]) 10. Januar 2013 (2013-01-10) * Ansprüche 1,6,19,24,38; Abbildung 2 * ----- | 7 | |
| A | | 1-6 | |
| A | BE 1 018 840 A3 (WASTE ENERGY RECOVERED [BE]) 6. September 2011 (2011-09-06) * Ansprüche 1-4 * ----- | 1-7 | |
| A,D | EP 1 772 202 A1 (SCHERRER INST PAUL [CH]) 11. April 2007 (2007-04-11) * Abbildungen 1,2 * ----- | 1-6 | |

-/--

Der vorliegende Recherchenbericht wurde für alle Patentansprüche erstellt

| Recherchenort | Abschlußdatum der Recherche | Prüfer |
|---|---|---|
| München | 26. Mai 2014 | Van Iddekinge, R |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes
    Dokument

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 14 15 9871

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | JP S61 66789 A (AGENCY IND SCIENCE TECHN) 5. April 1986 (1986-04-05) * Zusammenfassung * ----- | 1-6 | |
| A | YOSHIDA H ET AL: "Efficient, high-speed methane fermentation for sewage sludge using subcritical water hydrolysis as pretreatment", BIORESOURCE TECHNOLOGY, ELSEVIER BV, GB, Bd. 100, Nr. 12, 1. Juni 2009 (2009-06-01) , Seiten 2933-2939, XP026027142, ISSN: 0960-8524, DOI: 10.1016/J.BIORTECH.2009.01.047 [gefunden am 2009-02-28] * Zusammenfassung; Abbildung 1 * ----- | 1-7 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 26. Mai 2014 | Van Iddekinge, R |

**ANHANG ZUM EUROPÄISCHEN RECHERCHENBERICHT**
**ÜBER DIE EUROPÄISCHE PATENTANMELDUNG NR.**

EP 14 15 9871

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-05-2014

| Im Recherchenbericht angeführtes Patentdokument | Datum der Veröffentlichung | Mitglied(er) der Patentfamilie | Datum der Veröffentlichung |
|---|---|---|---|
| DE 10217165 A1 | 05-02-2004 | KEINE | |
| DE 20220307 U1 | 30-04-2003 | KEINE | |
| WO 2011154226 A1 | 15-12-2011 | KEINE | |
| DE 29913370 U1 | 23-09-1999 | KEINE | |
| US 5707417 A | 13-01-1998 | JP 2647804 B2<br>JP H0899099 A<br>US 5707417 A | 27-08-1997<br>16-04-1996<br>13-01-1998 |
| WO 2013005202 A1 | 10-01-2013 | KEINE | |
| BE 1018840 A3 | 06-09-2011 | KEINE | |
| EP 1772202 A1 | 11-04-2007 | BR PI0616850 A2<br>CA 2624510 A1<br>CN 101278033 A<br>EP 1772202 A1<br>EP 1931753 A1<br>JP 4977826 B2<br>JP 2009509759 A<br>US 2009126274 A1<br>WO 2007038996 A1 | 05-07-2011<br>12-04-2007<br>01-10-2008<br>11-04-2007<br>18-06-2008<br>18-07-2012<br>12-03-2009<br>21-05-2009<br>12-04-2007 |
| JP S6166789 A | 05-04-1986 | JP H0336871 B2<br>JP S6166789 A | 03-06-1991<br>05-04-1986 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1772202 A1

**In der Beschreibung aufgeführte Nicht-Patentliteratur**

- **VOGEL, F. ; F. HILDEBRAND.** Catalytic Hydrothermal Gasification of Woody Biomass at High Feed Concentrations. *Chem. Eng. Trans.,* 2002, vol. 2, 771-777